# EUROPEAN PATENT SPECIFICATION

(11) **EP 2 881 040 B1**
(45) Date of publication and mention of the grant of the patent: **27.07.2016**
(21) Application number: 14195212.7
(22) Date of filing: 27.11.2014
(51) Int. Cl.: A61B 6/14, A61B 6/02, A61B 6/00

(54) **Method and apparatus for acquiring a scout image in a digital dental radiographic apparatus**
Verfahren und Vorrichtung zur Erfassung eines Scout-Bildes in einer digitalen dentalen Radiografievorrichtung
Procédé et appareil pour acquérir une image informatrice dans un appareil de radiographie dentaire numérique

(30) Priority: 02.12.2013 IT BO20130669
(43) Date of publication of application: 10.06.2015
(73) Proprietor: CEFLA SOCIETA' COOPERATIVA, 40026 Imola (BO) (IT)
(72) Inventor: Manuzzato, Gianluca, 40026 Imola (BO) (IT); Bruno, Andrea, 40026 Imola (BO) (IT); Zoccatelli, Giacomo, 37024 Negrar (VA) (IT)
(74) Representative: Karaghiosoff, Giorgio Alessandro

(56) References cited:
- EP-A1- 2 614 773
- US-A1- 2009 168 966
- US-B2- 7 787 586

## Description

The present invention relates to dental radiographic apparatuses, known as panoramic apparatuses. In particular, the present invention relates to a method and an apparatus capable of performing a scout acquisition, preceding the acquisition of a real panoramic image, in a particularly efficient way.

Panoramic apparatuses produce a panoramic image of the dental arches of a patient, exposing a patient skull to X-rays. Panoramic radiography (also known as orthopantomography) is a radiographic image of a curved plan, also known as Welander curve, approximating patient jaws, with blurring of the anatomical structures laying outside a narrow layer around the predesigned curved plane. This technology has been known since the '50s; for the first 30-40 years (1950-1990) a film used to be exposed to X-rays. Nowadays such apparatuses use digital sensors, converting the X-rays hitting the X-ray detector into an electric signal, which suitably processed forms a digital image. The set of movements that mechanical parts have to perform to get this result is called trajectory.

Typically, a panoramic apparatus comprises an X-ray source and detector fixed to the ends of a rigid support, while the patient is positioned in an intermediate position between said X-ray source and detector. In the known art, apparatuses are known wherein the X-ray source and detector are moved according to pre-set trajectories thanks to the combination of three movements, i.e. a rotational movement around an axis of rotation of the rigid support thanks to which X-ray source and detector are moved along a circular trajectory around said axis and around the patient, and a translational movement of the axis of rotation of support arm according at least one or two different directions in the horizontal plane perpendicular to the axis of rotation of the support arm. Moreover, also apparatuses are known, wherein the displacement according to two directions in the plane perpendicular to the axis of rotation of the support arm occurs not in a translational way but in a rotational way, thanks to an angular transfer of the rotation axis of the support arm around an axis of rotation parallel to the rotation axis of the support arm itself, as described e.g. in WO2011064449A1 Planmeca.

A panoramic apparatus also comprises a patient positioning and immobilizing device, for holding the patient during the acquisition.

In the known art, in the normal working flow the human operator must perform some manual operations for setting the apparatus in order to get a good quality image: such operations comprise patient positioning, and the choice of technical exposure factors, up to the setting of additional options provided by the apparatus, in order to get a trajectory of X-ray source and detector of the panoramic apparatus during the acquisition that follows as accurately as possible the single patient's Welander curve.

Nowadays, the majority of manual operations is left to operator's experience and competence. Many apparatuses have devices helping the operator in performing manual operations: e.g. the apparatus proposes to the operator pre-defined settings of technical exposure factors, suitably adjusted according to patient's gender and size: such a system is described e.g. in US4618974 Siemens. However, the selection of these settings is always left to human operator. The system described in US4618974 is based on statistical criteria and on operator's competence.

A more precise way to adjust technical exposure factors (X-ray tube power (kV), X-ray tube current (mA), duration of exposure (sec) and film speed) and /or acquisition trajectory consists in performing a pre-acquisition of the single patient, known in the art as scout image.

In the art different kinds of scout image are known: a first kind acquires the whole area to be acquired, from which the operator then extracts a Region Of Interest (ROI); on the basis of ROI technical exposure factors are then set. A second way consists in acquiring a scout having reduced dimensions with respect to the area to be acquired, assuming that this small area is representative of the total area to be acquired, as described e.g. in US 7519155 Morita.

A further way of the known art consists in acquiring a patient's image in the visible range, to then adjust panoramic trajectory to the anatomy of that specific patient (face scan). A patent describing the use of a camera connected to a panoramic apparatus is e.g. Lemchen's US6081739.

The evolution of known art goes into the direction of making the adjustment of technical exposure factors and/or acquisition trajectory more and more automatic, and operator-independent. Therefore methods and devices automatically obtaining the necessary information from the scout image are designed.

For the success of automatisms and for better patient's comfort, the time interval between scout acquisition and panoramic real acquisition should be as short as possible, so as to prevent patient's movement between scout and real panoramic acquisition.

Two different problems may occur due patient's movement:
- In a first case, patient moves during real panoramic acquisition, producing motion artefacts;
- In a second case, patient stands still during scout acquisition and real panoramic acquisition, but moves between scout acquisition and real panoramic acquisition, so invalidating the assumption that the scout is representative of the area to be acquired during the real panoramic acquisition.

In the second case, one needs to get a scout of a broader area, administering a higher X-ray dose to patient. But one has always to keep into account that X-rays can lead to patient biological damage, as X-rays are ionizing radiations which can damage cell DNA. Therefore, the need to administer the lowest possible X-ray dose to the single patient under examination is apparent.

The present invention has the aim of performing a pre-acquisition imaging known as scout, such as that operator's intervention is kept to a minimum, the area acquired during scout acquisition is as small as possible, and that the acquisition of both scout image and real image is performed in as short as possible time, and with the best comfort for both operator and patient.

This object is achieved by a method and an apparatus having the features of the independent claims. Advantageous embodiments and refinements are specified in claims dependent thereon.

In the present invention a method and an apparatus are described wherein a scout acquisition of the single patient is performed immediately before the real panoramic acquisition: such scout typically has a much smaller dimension (1-8%) with respect to the area acquired during real panoramic acquisition. For acquiring the scout image, apparatus movement possibilities are exploited, in that scout acquisition occurs with a rotation direction of X-ray source and detector around the patient which is opposed to that of the real panoramic acquisition.

It is to be noted that there is a relation between acquisition time and dimension of the acquired area. As a matter of fact, considering a substantially constant speed of rotation of the X-ray source and detector around the virtual rotation centre (CVR), the temporal duration of the acquisition corresponds to a given angular displacement of X-ray source and detector around the patient, and therefore to a given dimension of the acquired area.

A first advantage of the present invention is the absence of additional steps for both operator and patient in the acquisition flow with respect to the known art.

A second advantage of the present invention is the minimization of the overall time for acquiring a panoramic image.

A third advantage of the present invention is the minimization of the probability of patient's motion between scout acquisition and real panoramic acquisition.

A fourth advantage of the present invention is the minimization and tailoring of X-ray dose administered to the single patient undergoing examination.

In the present specification and in the claims the term "virtual rotation centre (CVR) has the same meaning of instant centre of rotation in cinematic or also instantaneous centre of zero velocity (IC). Considering a rigid body experiencing a general plane motion (in two dimensions), the instant centre is an imaginary point lying on an imaginary axis of zero velocity about which a rigid body appears to rotate at a given instant and which axis is always perpendicular to the plane of motion. The instant centre is a limiting case of the so-called pole of planar rotation, which is the point around which any planar displacement of a body considered as a combination of a planar rotation and of a planar translation can be viewed as a rotation around this pole. If the initial and end position of the body are separated by an instant of time in a planar movement then the pole of displacement becomes an instant centre of rotation.

In the particular motion of the x-ray source and of the detector, the arm carrying the x-ray source and the detector is displaced by a roto-translational movement in such a way as to displace the source and the detector along non-circular paths (Welander curve). Thus the instant centre of rotation changes its position in space for different segments of the said path for the source and the detector. (the path of the instant centre of rotation in time is called centrode).

Further advantages and properties of the present invention are disclosed in the following description, in which exemplary embodiments of the present invention are explained in detail based on the drawings:
Figure 1 Example of panoramic apparatus;
Figure 2 Movement steps of the rotating arm supporting X-ray source and detector during the acquisition of an overall scout+panoramic image according to the present invention;
Figure 3 Movements of said rotating arm in a full-frame acquisition system;
Figure 4 Movements of said rotating arm in a TDI acquisition system;
Figure 5 Movements of said rotating arm which are possible by shifting the virtual centre of rotation;
Figure 6 Movement steps of rotating arm during the acquisition of an overall scout+panoramic image in a TDI acquisition system according to the present invention.

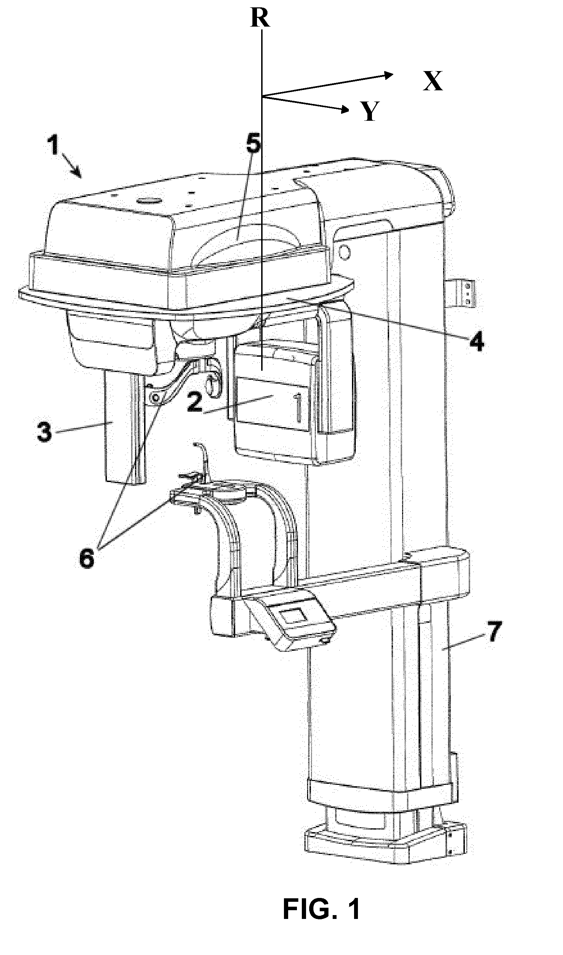

Figure 1 shows a typical panoramic radiographic apparatus 1, comprising an X-ray source 2 projecting a collimated X-ray bundle across a (not shown) patient, a bi-dimensional X-ray detector 3 positioned so as to measure the intensity of radiation after it crossed the patient, a C-arm 4 on which said X-ray source 2 and detector 3 are fixed, a mechanical system 5 rotating and translating said support C-arm 4 around the patient so that said C-arm moves said X-ray source and detector around the patient, so as to acquire radiographic images from different positions.

In the present description the words detector or sensor are equivalent, meaning a unit receiving the radiation transmitted through the patient, and transforming the intensity of said radiation into electric signals corresponding to the intensity of said radiation; the mechanical system 5 is provided with at least two, possibly three, different degrees of freedom of movement, which allow the rotation of C-arm 4 around an axis R perpendicular to the longitudinal extension of the arm, and intermediate between X-ray source 2 and detector 3, and the translation of said axis of rotation R according to at least one, possibly two, different directions X, Y in a plane perpendicular to said axis of rotation R of C-arm 4. Panoramic apparatus 1 comprises also a (not shown) electronic system to control and synchronize the working of the various apparatus components. Moreover, panoramic apparatus 1 comprises a device 6 for positioning the patient.

In the non-limiting example of Figure 1, the patient positioning device 6 consists of a bite and supports for positioning patient's skull. The position of C-arm 4 can be adjusted to patient's height thanks to vertically mobile post 7.

Generally, in today's digital panoramic apparatuses, before exposure, the rotating group, consisting of C-arm 4 and X-ray source 2 and detector 3, is positioned in the so-called angular "patient entry" position, i.e. the position wherein, according to the mechanical structure of the apparatus, patient entry into the compartment below the arm between X-ray source and detector, and the access into patient's positioning device 6 are allowed.

In the present exemplary embodiment, this position is reached positioning C-arm 4 with its longitudinal axis perpendicular to patient positioning device 6, i.e. to the anteroposterior axis of patient's skull positioned in said patient positioning device 6, in order to allow a comfortable access of patient to positioning device.

From this position, which can be called RESET, C-arm 4 must move to "EXAM START" position, i.e. to the point wherein X-ray emission starts, with the consequent acquisition of patient radiographic image; in most exams these two positions (RESET and EXAM START) do not coincide.

In a panoramic acquisition, in particular, image acquisition and therefore exposure starts while the rotating arm 4 is positioned so that X-ray sensor 3 is behind patient's condyle, and X-ray tube, i.e. X-ray generator 2, is angularly displaced of about 30°--55° with respect to RESET position.

For good ergonomics, the scout acquisition should be performed so that the trajectory of rotating arm 4 during scout acquisition starts from RESET point and ends in EXAM START position.

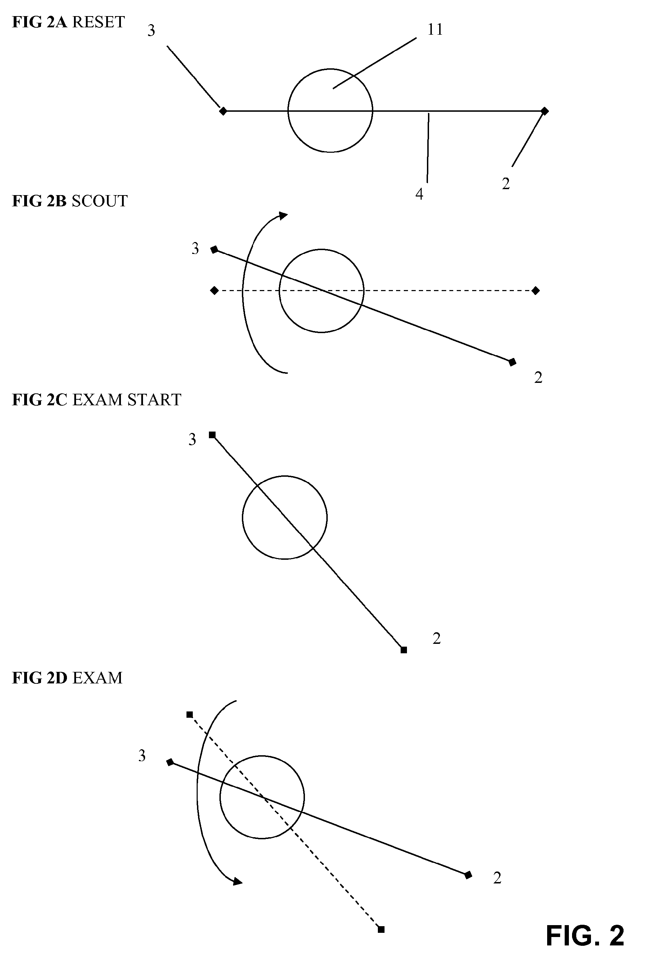

Figure 2 shows in a very stylized way X-ray source 2 and digital bi-dimensional X-ray detector 3 united by C-arm 4; the whole rotates around patient 11.

Figure 2A shows the typical RESET position. From here the assembly X-ray source 2, detector 3 and C-arm 4 of apparatus 1 must go to the position EXAM START shown in Figure C, passing through the position shown in Figure 2B.

In Figure 2B the sense of C-arm 4 rotation is clockwise, as shown by the curved arrow. C-arm 4 rotates angularly displacing X-ray source 2 and detector 3 around patient 11 to bring the C-arm 4 and said X-ray source 2 and detector 3 from position 2A to position 2C. The heart of invention lies in exploiting the movement shown in Figure 2B to acquire scout image.

Figure 2D shows the acquisition of the real panoramic image, performed in a direction opposed to that of the scout acquisition. In the present embodiment, the real acquisition is performed anticlockwise, as shown by the curved arrow. In Figure 2B the dotted line indicates the position of C-arm 4 in RESET position, and therefore allows to visualize the movement that C-arm 4 performed during scout acquisition. In Figure 2D the dotted line indicates the position of C-arm 4 at exam start, and allows therefore to visualize the movement that C-arm 4 performs during the acquisition of the real panoramic image.

Any type of bi-dimensional digital detector can be represented as an ordered sets of columns, each formed by X-ray photosensitive elements.

The two main acquisition technologies known in the art are:
- full-frame systems, typical, but not exclusive, of C-MOS detectors

In full-frame systems, all detector columns are exposed at the same time, and at pre-set periods of time are read all together, according to a given order. Once they have been read, their information content is cancelled. The final image is obtained reconstructing the information read in each frame.
- Time-Delay Integration (TDI), typical, but not exclusive, of CCD detectors

In TDI systems all detector columns are exposed at the same time, and at pre-set period of times only one of the two columns at the ends of the detector is read, which is called read-out column. The information content of all non-read columns is shifted to the adjacent column on the right or on the left, depending on detector setting, summing it up to information content already present in that column. In this way detector columns, during the shifting between the first position and read-out position, gather information content minimizing the quantity of X-ray dose administered to patient. The final image is obtained reconstructing the information read in each column.

In the following, two different embodiments will be described, each using a different bi-dimensional digital detector, C-MOS and CCD, whose implementation poses different challenges of realization.

The first embodiment makes use of full-frame technology, coupled with a C-MOS detector. Although this methodology of acquisition is more onerous under the point of view of both hardware and final image reconstruction, it does not pose limitations neither in the read-out direction of columns, and therefore nor in the rotation direction of the system around patient.

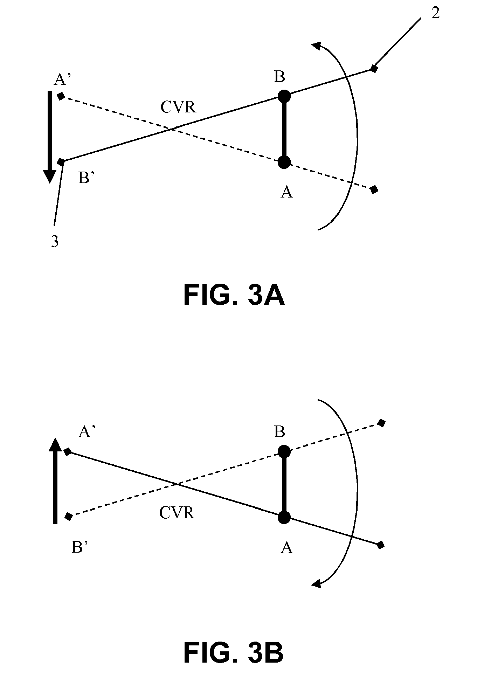

In Figure 3A a small portion of an acquisition is shown. As known, a panoramic is formed by successive acquisitions performed by the digital detector, which are then processed to provide the image of a whole patient's arch. In Figure 3A, the dotted line indicates the starting position of C-arm 4 at time t0, while the continuous line indicates the successive position of C-arm 4 at time t1. Between t0 and t1 the acquisition of a small anatomic portion occurs, represented by segment AB, which is projected on sensor 3 in the positions A' and B', respectively.

The curved arrow indicates the rotation direction of C-arm 4, while the bold linear arrow indicates the reading direction of detector, which must be coherent with the rotation direction of exposure.

Since C-arm 4 of the present invention has three degrees of freedom of movement, i.e. rotation around axis R and translation in the two directions X, Y, the virtual centre of rotation, represented in the Figure with CVR, does not necessarily correspond to the axis of rotation R of C-arm 4, but the said centre of rotation R can be translated during its rotation in the plane perpendicular to rotation axis in the directions X, Y. This leads to the formation of a virtual centre of rotation CVR for all or part of overall acquisition trajectory. System controlling two or more axes can generally move CVR during acquisition so as to obtain complex trajectories.

Figure 3B shows the small portion of acquisition of Figure 3A, wherein the direction of rotation and direction of detector reading are inverted with respect to Figure 3A, as shown by the curved arrow.

Here the term "virtual center of rotation" (CVR) has the meaning of instant center of rotation as already defined above.

Full-frame technology for C-MOS systems allows to read detectors indifferently in both directions, and therefore this embodiment does not have particular difficulties in its implementation.

The second embodiment makes use of TDI technology coupled with a CCD X-ray detector.

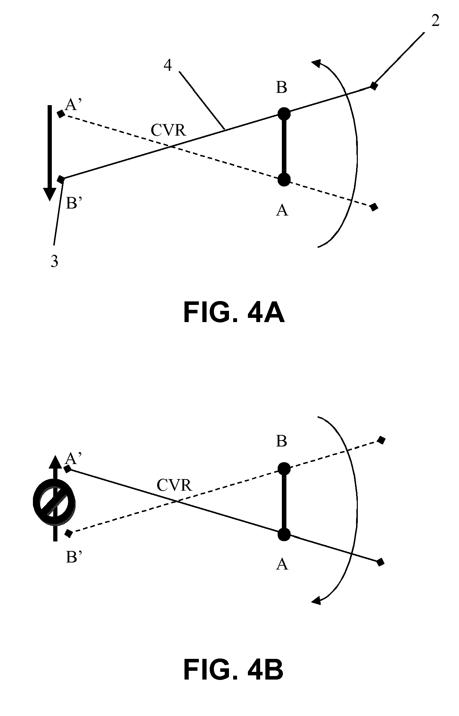

With respect to the first embodiment using a C-MOS detector, the read-out direction of a CCD detector is pre-set by the manufacturer of the detector. In order to acquire the same AB object by inverting the direction of trajectory, artifices must be used for maintaining the same read-out direction of detector, while performing the rotation for scout acquisition in the direction opposite to pre-set reading direction of detector. Figures 4A and 4B show that one reading direction is possible, the opposite is not.

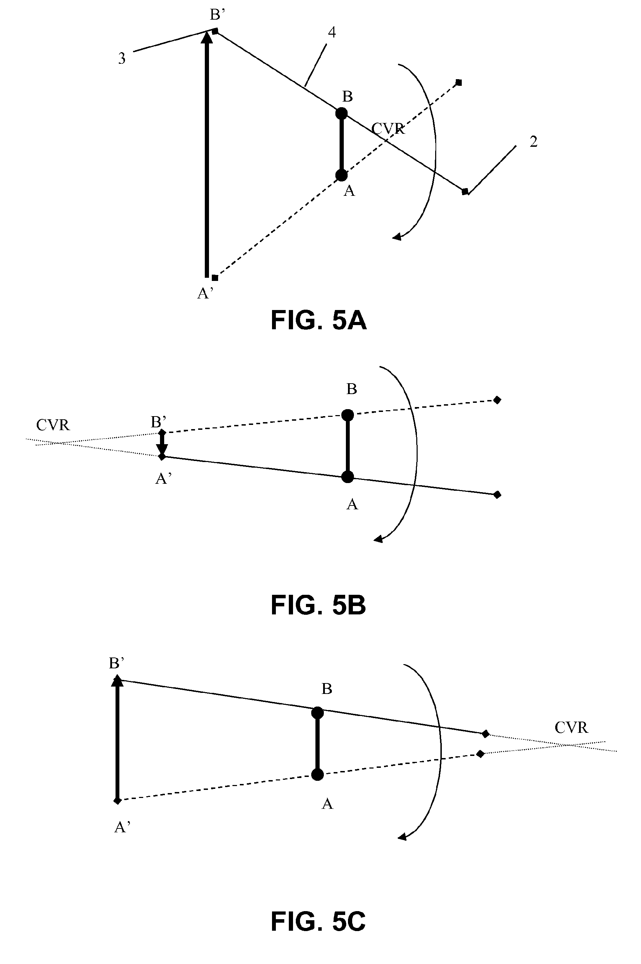

The artifice consists in using trajectories of movement of the assembly comprising C-arm 4, X-ray source 2 and detector 3 wherein the virtual centre of rotation CVR shifts with respect to the position shown in Figure 4A. Different configurations are possible, which are shown in Figures 5A, 5B, 5C.

Figure 5A shows the shifting of position of the virtual centre of rotation (CVR) beyond object (AB), but in a point situated between the object and X-ray source 2.

Figure 5B shows the shifting of the position of the virtual centre of rotation (CVR) beyond the detector (A'B').

Figure 5C shows the shifting of the position of the virtual centre of rotation (CVR) beyond object (AB), out of the interval X-ray detector-source.

The configurations shown in Figure 5A and 5C are of purely theoretical interest, in that the reading direction of detector is still "forbidden".

Instead, the configuration shown in Figure 5B is usable in that the read-out direction of detector continues to be the in pre-set direction of the detector. Moreover, in the configuration shown in Figure 5B, the final arm 4 position is the most compatible with the position of exam start.

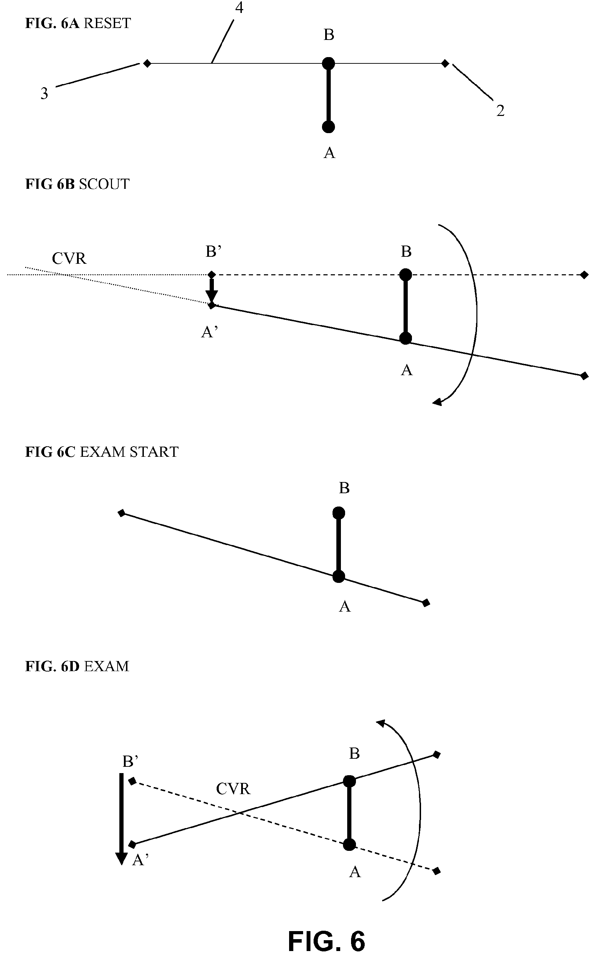

The acquisition of a whole panoramic image is shown in Figure 6.

During scout acquisition a complex trajectory is performed, wherein the virtual centre of rotation moves in the neighbourhood of point CVR shown in Figure 6B, while during the acquisition of the real panoramic image the virtual centre of rotation describes a complex trajectory in the neighbourhood of point CVR shown in Figure 6D. As it is apparent in comparing Figures 6B and 6D, during scout acquisition CVR is beyond X-ray sensor 3, on the opposite side to X-ray source 2, while during real panoramic acquisition CVR is between the object to be acquired and X-ray sensor 3.

It is apparent that the definition of the virtual centre of rotation is not free from consequences on the magnifying factor of the image, and therefore the different magnifying factors will have to be taken into account in the scout with respect to the real acquisition, or positions of CVR will have to be chosen so as to have the same magnification.

The setting of the virtual centre of rotation (CVR) according to the configuration of Figure 5B allows to bring back the acquisition of scout and real image to what is shown in Figure 2, i.e. the direction of scout acquisition is opposed with respect to acquisition direction of real panoramic image.

Basically, Figure 6 corresponds to Figure 2, wherein the acquisition is partitioned in four stages t0, t1, t2, t3 and therefore the object to be acquired is not the whole dental arch indicated in Figure 2 with 11, but the successive small anatomic portions AB acquired at each time by detector 3.

In Figure 6 it is apparent that each final point of C-arm 4 in a given step, which in the figures is indicated with dotted line, corresponds to the starting point of the successive step. As a consequence, all the steps (RESET, SCOUT, EXAM START and EXAM) can be performed one after the other, minimizing the acquisition time of the whole panoramic image, formed by scout plus real acquisition.

The present description always referred to the preferred embodiment, for the acquisition of a panoramic image of a patient; it is however apparent to the skilled person that this method for acquiring a scout before the acquisition of a real panoramic image can be advantageously used also for acquisitions according to different trajectories, e.g. performing acquisitions on condyles, maxillary sinuses, small arch portions.

Moreover, the same method can be used in cone beam apparatuses for the acquisition of volumetric three-dimensional images (Cone-Beam Computerized Tomography, CBCT).

## Claims

1. Method for acquiring a radiographic image of a patient with an apparatus comprising at least an X-ray source and at least an X-ray detector, said X-ray source and detector being supported in an opposed position, the objet under investigation being positioned in an intermediate position between said X-ray source and detector and in the propagation bundle of the radiation emitted by said X-ray source, moreover said X-ray source and detector being movable along a pre-set trajectory thanks to at least two movements:
- a rotational movement of said X-ray source and detector around a rotation axis (R),
- a translational movement of said axis of rotation (R) along at least a direction (Y) in the horizontal plane perpendicular to said rotation axis (R), or a rotational movement of said rotation axis (R) around a second axis of rotation perpendicular to the horizontal plane, i.e. parallel to said common rotation axis (R);
while thanks to the combination of said movements, the virtual rotation centre (CVR), i.e. the instant center of rotation, of said X-ray source and detector moves during each acquisition, the method comprising:
- the acquisition of a pre-exam image (scout)
- the successive acquisition of the real image,
**characterized in that** for acquiring said pre-exam image, the rotation of said X-ray source and detector around the common axis of rotation (R) occurs in opposite direction with respect to the rotation direction of said X-ray source and detector around the common rotation axis (R) during the exposure for acquiring the real image.

2. Method for acquiring a radiographic image of a patient according to claim 1, wherein X-ray source and detector perform three combined movements, i.e. a rotational movement around the common rotation axis (R), a translational movement in the plane perpendicular to the common axis of rotation (R) along at least a first direction (X), a further translational movement in the plane perpendicular to the common axis of rotation (R) along at least a second direction (Y) different from the first, or a combined translational movement according to two different directions along a curved line thanks to a rotational movement of said axis of rotation (R) around to said second axis of rotation parallel to the common axis of rotation (R) itself.

3. Method for acquiring a radiographic image of a patient according to claim 1 or 2, wherein the acquisition of the real image occurs immediately after scout acquisition, without repositioning said patient.

4. Method for acquiring a radiographic image of a patient according to claim 1or 2, wherein image acquisition occurs with full-frame technology.

5. Method for acquiring a radiographic image of a patient according to claim 1 or 2, wherein image acquisition occurs with Time-Delay Integration technology (TDI).

6. Method for acquiring a radiographic image of a patient according to claim 5, wherein scout acquisition and real image acquisition occur each with the virtual rotation centre (CVR) in a relative different position with respect to the group comprising X-ray source, detector and patient under investigation.

7. Method for acquiring a radiographic image of a patient according to claim 6, wherein the virtual centre of rotation during the scout acquisition is beyond X-ray detector on the side opposed to X-ray source.

8. Method for acquiring a radiographic image of a patient according to claim 1-7, wherein a bi-dimensional image or a volumetric three-dimensional image of a patient's maxilla and mandible is acquired.

9. Apparatus (1) for acquiring radiographic images comprising an X-ray source (2) projecting a collimated X-ray bundle across a patient (11), a bi-dimensional X-ray detector (3) positioned so as to measure the intensity of radiation after it crossed the patient, a C-arm (4) on which said X-ray source (2) and detector (3) are fixed, said C-arm (4) being provided with at least two movements, the first being a rotational movement around the axis of rotation around (R), and the at least second a translational movement of the axis of rotation (R) along a direction (Y) in the horizontal plane perpendicular to said axis of rotation (R) or another rotational movement of the rotation axis (R) of C-arm (4) around a second axis of rotation; the position of C-arm (4) being adjusted to patient's height thanks to vertically mobile post (7); a mechanical system (5) so as to allow rotation and translation of said C-arm around the patient, so as to acquire radiographic images from different positions; a device 6 for positioning the patient; an (not shown) electronic system controlling and synchronizing the working of the various components of the apparatus
**characterized in that** it is adapted to perform the method of claims 1-8.

10. Apparatus for acquiring radiographic images according to claim 9, moreover comprising means for movement according to a translational movement of said axis of rotation (R) of C-arm (4) along a second direction of translation (X) in the horizontal plane perpendicular to the axis of rotation (R) of C-arm (4), the second direction being different from said first direction of translation (Y), or a mechanism for translating said axis of rotation (R) of said C-arm (4) according to a rotational movement around an axis of rotation parallel to axis of rotation (R).

## Patentansprüche

1. Verfahren zum Aufnehmen eines radiografischen Bildes eines Patienten mit einer Vorrichtung, die mindestens eine Röntgenquelle und mindestens einen Röntgendetektor umfasst, wobei die Röntgenquelle und der Röntgendetektor in einer einander gegenüberliegenden Position gestützt werden, wobei der untersuchte Proband in einer Position zwischen der Röntgenquelle und dem Röntgendetektor und in dem Ausbreitungsbündel der durch die Röntgenquelle ausgesendeten Strahlung positioniert ist, wobei darüber hinaus die Röntgenquelle und der Röntgendetektor dank mindestens zweier Bewegungen entlang eines voreingestellten Bewegungspfades bewegt werden können:
□ eine Rotationsbewegung der Röntgenquelle und des Röntgendetektors um eine Rotationsachse (R),
□ eine Translationsbewegung der Rotationsachse (R) entlang mindestens einer Richtung (Y) in der horizontalen Ebene senkrecht zu der Rotationsachse (R) oder eine Rotationsbewegung der Rotationsachse (R) um eine zweite Rotationsachse senkrecht zu der horizontalen Ebene, d. h. parallel zu der gemeinsamen Rotationsachse (R);
während sich dank der Kombination der Bewegungen der virtuelle Rotationsmittelpunkt (CVR), d. h. der augenblickliche Rotationsmittelpunkt, der Röntgenquelle und des Röntgendetektors während jeder Aufnahme bewegt, wobei das Verfahren Folgendes umfasst:
□ die Aufnahme eines Voruntersuchungsbildes (Scout)
□ die nachfolgende Aufnahme des realen Bildes,
**dadurch gekennzeichnet, dass** für die Aufnahme des Voruntersuchungsbildes die Rotation der Röntgenquelle und des Röntgendetektors um die gemeinsame Rotationsachse (R) in entgegengesetzter Richtung mit Bezug auf die Rotationsrichtung der Röntgenquelle und des Röntgendetektors um die gemeinsame Rotationsachse (R) während der Belichtung für die Aufnahme des reale Bildes erfolgt.

2. Verfahren zum Aufnehmen eines radiografischen Bildes eines Patienten nach Anspruch 1, wobei die Röntgenquelle und der Röntgendetektor drei kombinierte Bewegungen ausführen, und zwar eine Rotationsbewegung um die gemeinsame Rotationsachse (R), eine Translationsbewegung in der Ebene senkrecht zu der gemeinsamen Rotationsachse (R) entlang mindestens einer ersten Richtung (X), eine weitere Translationsbewegung in der Ebene senkrecht zu der gemeinsamen Rotationsachse (R) entlang mindestens einer zweiten Richtung (Y), die von der ersten verschieden ist, oder eine kombinierte Translationsbewegung gemäß zweier verschiedener Richtungen entlang eines gekrümmten Linie dank einer Rotationsbewegung der Rotationsachse (R) um die zweite Rotationsachse parallel zu der gemeinsamen Rotationsachse (R) selbst.

3. Verfahren zum Aufnehmen eines radiografischen Bildes eines Patienten nach Anspruch 1 oder 2, wobei die Aufnahme des realen Bildes unmittelbar nach der Scout-Aufnahme ohne Umpositionierung des Patienten stattfindet.

4. Verfahren zum Aufnehmen eines radiografischen Bildes eines Patienten nach Anspruch 1 oder 2, wobei die Bildaufnahme mit Vollbildtechnologie stattfindet.

5. Verfahren zum Aufnehmen eines radiografischen Bildes eines Patienten nach Anspruch 1 oder 2, wobei die Bildaufnahme mit Time-Delay Integration (TDI)-Technologie stattfindet.

6. Verfahren zum Aufnehmen eines radiografischen Bildes eines Patienten nach Anspruch 5, wobei die Scout-Aufnahme und die reale Bildaufnahme jeweils erfolgen, während sich der virtuelle Rotationsmittelpunkt (CVR) in einer relativ anderen Position mit Bezug auf die Gruppe bestehend aus Röntgenquelle, Röntgendetektor und untersuchtem Patienten befindet.

7. Verfahren zum Aufnehmen eines radiografischen Bildes eines Patienten nach Anspruch 6, wobei der virtuelle Rotationsmittelpunkt während der Scout-Aufnahme jenseits des Röntgendetektors auf der Seite gegenüber der Röntgenquelle liegt.

8. Verfahren zum Aufnehmen eines radiografischen Bildes eines Patienten nach den Ansprüchen 1 bis 7, wobei ein zweidimensionales Bild oder ein volumetrisches dreidimensionales Bild des Oberkiefers und des Unterkiefers eines Patienten aufgenommen wird.

9. Vorrichtung (1) zum Aufnehmen radiografischer Bilder, die Folgendes umfasst: eine Röntgenquelle (2), die ein kollimiertes Röntgenstrahlenbündel auf einen Patienten (11) projiziert, einen zweidimensionalen Röntgendetektor (3), der so positioniert ist, dass er die Intensität von Strahlung misst, nachdem sie den Patienten passiert hat, einen C-Arm (4), auf dem die Röntgenquelle (2) und der Röntgendetektor (3) befestigt werden, wobei der C-Arm (4) mit mindestens zwei Bewegungen versehen ist, wobei die erste eine Rotationsbewegung um die Rotationsachse (R) ist und die mindestens zweite eine Translationsbewegung der Rotationsachse (R) entlang einer Richtung (Y) in der horizontalen Ebene senkrecht zu der Rotationsachse (R) oder eine weitere Rotationsbewegung der Rotationsachse (R) des C-Arms (4) um eine zweite Rotationsachse ist; wobei die Position des C-Arms (4) dank des vertikal beweglichen Pfostens (7) auf die Körpergröße des Patienten eingestellt wird; ein mechanisches System (5), um eine Rotation und Translation des C-Arms um den Patienten zu ermöglichen, um radiografische Bilder aus verschiedenen Positionen aufzunehmen; eine Vorrichtung 6 zur Positionierung des Patienten; ein (nicht gezeigtes) elektronisches System zum Steuern und Synchronisieren der Funktion der verschiedenen Komponenten der Vorrichtung,
**dadurch gekennzeichnet, dass** sie dafür ausgelegt ist, das Verfahren der Ansprüche 1 bis 8 auszuführen.

10. Vorrichtung zum Aufnehmen radiografischer Bilder nach Anspruch 9, die des Weiteren ein Mittel für eine Bewegung gemäß einer Translationsbewegung der Rotationsachse (R) des C-Arms (4) entlang einer zweiten Translationsrichtung (X) in der horizontalen Ebene senkrecht zu der Rotationsachse (R) des C-Arms (4) umfasst, wobei die zweite Richtung von der ersten Translationsrichtung (Y) verschiedenen ist, oder einen Mechanismus zum Translatieren der Rotationsachse (R) des C-Arms (4) gemäß einer Rotationsbewegung um eine Rotationsachse parallel zu der Rotationsachse (R) umfasst.

## Revendications

1. Procédé d'acquisition d'une image radiographique d'un patient au moyen d'un appareil comprenant au moins une source de rayons X et au moins un détecteur de rayons X, ladite source et ledit détecteur de rayons X étant portés dans une position intermédiaire entre ladite source et ledit détecteur de rayons X et à l'intérieur du faisceau de propagation de la radiation émise par ladite source de rayons X, ladite source et ledit détecteur de rayons X étant par ailleurs mobiles le long d'une trajectoire prédéfinie selon au moins deux mouvements :
- un mouvement de rotation de ladite source et dudit détecteur de rayons X autour d'un axe de rotation (R),
- un mouvement de translation dudit axe de rotation (R) le long d'au moins une direction (Y) dans le plan horizontal perpendiculaire audit axe de rotation (R), ou un mouvement de rotation dudit axe de rotation (R) autour d'un deuxième axe de rotation perpendiculaire au plan horizontal, c'est-à-dire parallèle audit axe de rotation commun (R) ;
alors que, grâce à la combinaison desdits mouvements, le centre virtuel de rotation (CVR), c'est-à-dire le centre instantané de rotation de ladite source et dudit détecteur de rayons X se déplace pendant chaque acquisition, le procédé comprenant :
- l'acquisition d'une image pré-examen (topogramme)
- l'acquisition successive de l'image réelle,
**caractérisé en ce que**, pour acquérir ladite image pré-examen, la rotation de ladite source et dudit détecteur de rayons X autour de l'axe de rotation commun (R) a lieu dans le sens opposé au sens de rotation de ladite source et dudit détecteur de rayons X autour de l'axe de rotation commun (R) pendant l'exposition pour l'acquisition de l'image réelle.

2. Procédé d'acquisition d'une image radiographique d'un patient selon la revendication 1, dans lequel la source et le détecteur de rayons X effectuent trois mouvements combinés, c'est-à-dire un mouvement de rotation autour de l'axe de rotation commun (R), un mouvement de translation dans le plan perpendiculaire à l'axe de rotation commun (R) le long d'au moins une première direction (X), un autre mouvement de translation dans le plan perpendiculaire audit axe de rotation commun (R) le long d'au moins une deuxième direction (Y) différente de la première, ou un mouvement de translation combiné selon deux directions différentes le long d'une ligne courbe grâce à un mouvement de rotation dudit axe de rotation (R) autour dudit deuxième axe de rotation parallèle à l'axe de rotation commun (R).

3. Procédé d'acquisition d'une image radiographique d'un patient selon la revendication 1 ou 2, dans lequel l'acquisition de l'image réelle a lieu immédiatement après l'acquisition du topogramme, sans repositionner ledit patient.

4. Procédé d'acquisition d'une image radiographique d'un patient selon la revendication 1 ou 2, dans lequel l'acquisition d'image a lieu avec la technologie plein cadre.

5. Procédé d'acquisition d'une image radiographique d'un patient selon la revendication 1 ou 2, dans lequel l'acquisition d'image a lieu avec la technologie d'intégration de lignes décalées dans le temps (Time-Delay Integration, TDI).

6. Procédé d'acquisition d'une image radiographique d'un patient selon la revendication 5, dans lequel l'acquisition du topogramme et l'acquisition de l'image réelle ont lieu chacune avec le centre virtuel de rotation (CVR) dans une position relative différente par rapport au groupe comprennent la source de rayons X, le détecteur et le patient examiné.

7. Procédé d'acquisition d'une image radiographique d'un patient selon la revendication 6, dans lequel le centre virtuel de rotation pendant l'acquisition du topogramme se trouve au-delà du détecteur de rayons X sur le côté opposé à la source de rayons X.

8. Procédé d'acquisition d'une image radiographique d'un patient selon les revendications 1 à 7, dans lequel une image bidimensionnelle ou une image tridimensionnelle volumétrique d'une mâchoire ou d'une mandibule est acquise.

9. Appareil (1) pour l'acquisition d'images radiographiques comprenant une source de rayons X (2) projetant un faisceau de rayons X collimé à travers un patient (11), un détecteur de rayons X bidimensionnel (3) positionné de manière à pouvoir mesurer l'intensité de la radiation une fois que celle-ci a traversé le patient, un bras en C (4) sur lequel ladite source (2) et ledit détecteur (4) de rayons X sont fixés, ledit bras en C pouvant effectuer au moins deux mouvements, le premier étant un mouvement de rotation autour de l'axe de rotation (R), et l'au moins deuxième mouvement étant un mouvement de translation de l'axe de rotation (R) le long d'une direction (Y) dans le plan horizontal perpendiculaire audit axe de rotation (R) ou un autre mouvement de rotation de l'axe de rotation (R) du bras en C (4) autour d'un deuxième axe de rotation ; la position du bras en C (4) étant réglée selon la taille du patient au moyen d'un montant (7) mobile verticalement (7) ; un système mécanique (5) pour permettre la rotation et la translation dudit bras en C autour du patient, de manière à acquérir des images radiographiques depuis des positions différentes ; un dispositif 6 de positionnement du patient ; un système électronique (non montré) contrôlant et synchronisant les opérations des éléments différents de l'appareil
**caractérisé en ce que**
il est conçu pour mettre en oeuvre le procédé selon les revendications 1 à 8.

10. Appareil pour l'acquisition d'images radiographiques selon la revendication 9, comprenant également des moyens de déplacement selon un mouvement de translation dudit axe de rotation (R) du bras en C (4) le long d'une deuxième direction de translation (X) dans le plan horizontal perpendiculaire à l'axe de rotation (R) du bras en C (4), la deuxième direction étant différente de ladite première direction de translation (Y), ou un mécanisme de translation dudit axe de rotation (R) dudit bras en C (S) selon un mouvement de rotation autour d'un axe de rotation parallèle à l'axe de rotation (R).
